(19) Europäisches Patentamt
European Patent Office
Office européen des brevets

(11) Veröffentlichungsnummer : **0 008 096**
**B1**

(12) # EUROPÄISCHE PATENTSCHRIFT

(45) Veröffentlichungstag der Patentschrift : **08.07.81**

(51) Int. Cl.³ : **C 07 C 29/124**, C 07 C 31/38

(21) Anmeldenummer : **79102805.3**

(22) Anmeldetag : **04.08.79**

(54) **Verfahren zur Herstellung von 2-(Perfluoralkyl)-äthanolen.**

(30) Priorität : **09.08.78 DE 2834795**

(43) Veröffentlichungstag der Anmeldung : **20.02.80 (Patentblatt 80/04)**

(45) Bekanntmachung des Hinweises auf die Patenterteilung : **08.07.81 Patentblatt 81/27**

(84) Benannte Vertragsstaaten : **BE DE FR GB IT NL**

(56) Entgegenhaltungen :
**FR - A - 2 180 113**

**CHEMICAL ABSTRACTS, Vol. 86, Nr. 23, 6 Juni 1977, Columbus, Ohio, USA.**
**MATSUO MASASHI et al. : "A new santhetic method for perfluoroalkylethyl alcohol", Zusammenfassung Nr. 170772w.**

(73) Patentinhaber : **HOECHST AKTIENGESELLSCHAFT**
**Zentrale Patentabteilung Postfach 80 03 20**
**D-6230 Frankfurt/Main 80 (DE)**

(72) Erfinder : **Schwenk, Ulrich, Dr.**
**Robert-Koch-Strasse 209**
**D-8263 Burghausen/Salzach (DE)**
Erfinder : **König, Inge**
**Luitpoldallee 13**
**D-8261 Mühldorf (DE)**
Erfinder : **Streitberger, Horst**
**Josef-Hofmiller-Weg 9**
**D-8262 Altötting (DE)**

**EP 0 008 096 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Verfahren zur Herstellung von 2-(Perfluoralkyl)-äthanolen

Die Erfindung betrifft ein Verfahren zur Herstellung von 2-(Perfluoralkyl)-äthanolen (im folgenden als $R_f$-äthanole bezeichnet) der allgemeinen Formel

$$R_fCH_2CH_2OH,$$

in der $R_f$ einen Perfluoralkylrest von 1 bis 21 Kohlenstoffatomen, der geradkettig oder endständig methylverzweigt ist, darstellt, durch Umsetzung der entsprechenden 2-(Perfluoralkyl)-äthyljodide (im folgenden: $R_f$-äthyljodide) mit Wasser und Amiden.

Es sind bereits verschiedene Verfahren zur Herstellung von $R_f$-äthanolen aus den entsprechenden 2-(Perfluoralkyl)-äthyljodiden bekannt.

Die bekannten Verfahren sind jedoch umständlich und aufwendig und führen erst über mehrere Stufen zu den gewünschten $R_f$-äthanolen.

So werden gemäß US-Patent 3 246 030 die $R_f$-äthyljodide zunächst in die entsprechenden Nitrate übergeführt, aus denen durch reduktive Spaltung, zum Beispiel mit Lithium-Aluminiumhydrid, $R_f$-äthanole erhalten werden.

Der deutschen Patentschrift 1 214 660 zufolge werden $R_f$-äthyljodide mit Oleum zu den entsprechenden Schwefelsäureestern umgesetzt und diese anschließend zu den $R_f$-äthanolen verseift.

Nach dem Verfahren der DE-OS 2 318 941 erhält man aus der Reaktion der $R_f$-äthyljodide mit Amiden der allgemeinen Formel RCONR'R", in denen R, R' und R" Wasserstoffatome oder niedere Alkylreste bedeuten, und Wasser Gemische aus $R_f$-äthanolen, 2-(Perfluoralkyl)-äthylestern ($R_f$-äthylestern ; verestert mit der dem verwendeten Amid zugrundeliegenden Säure) und 2-(Perfluoralkyl)-äthylenen ($R_f$-äthylenen).

Die $R_f$-äthylester müssen dann durch Verseifung oder Umesterung in $R_f$-äthanole übergeführt werden. Daneben entstehen noch weitere Nebenprodukte, die zum Teil, da sie wasserlöslich sind, durch Waschen mit Wasser entfernt werden können. Dies trifft jedoch nicht für die sich bis zu einer Menge von 5 Gew.-% bildenden 2-(Perfluoralkyl)-äthylamine ($R_f$-äthylamine) zu, die bei bestimmten Verwendungen der $R_f$-äthanole stören und daher entfernt werden müssen. Dies ist jedoch bei Gemischen, die Perfluoralkylreste verschiedener Kettenlänge aufweisen, wegen der unmittelbar benachbarten Siedepunkte auf destillativem Weg nicht möglich, sondern kann nur umständlich, beispielsweise über Ionenaustauscher oder die intermediäre Bildung der Hydrochloride erreicht werden. Das außerdem bei der Verwendung von Dimethylformamid beobachtete Auftreten von freier Ameisensäure führt wegen deren korrosiven Eigenschaften zu apparativen Schwierigkeiten. Außerdem wird die destillative Wiedergewinnung von Dimethylformamid durch Bildung eines Azeotrops mit Ameisensäure stark erschwert.

Es ist daher Aufgabe der vorliegenden Erfindung, ein Verfahren zu schaffen, mit dem die gewünschten $R_f$-äthanole mit hoher Ausbeute und ohne wesentliche Mengen störender Nebenprodukte in wirtschaftlicher Weise erhalten werden können.

Diese Aufgabe wird gelöst durch ein Verfahren der eingangs genannten Art, das dadurch gekennzeichnet ist, daß man als Amid N-Methyl-2-pyrrolidon einsetzt.

Man erzielt nach dem erfindungsgemäßen Verfahren einen praktisch vollständigen Umsatz der $R_f$-äthyljodide und erhält die gewünschten $R_f$-äthanole in hoher Ausbeute (85 % und mehr, bezogen auf eingesetztes $R_f$-äthyljodid). $R_f$-äthylester werden nicht gebildet. Basische, stickstoffhaltige Verbindungen entstehen nur in untergeordneten Mengen und können durch Waschen mit Wasser entfernt werden, so daß in den destillierten $R_f$-äthanolen keine stickstoffhaltigen Reaktionsprodukte auffindbar sind.

Die als Ausgangsmaterial verwendeten $R_f$-äthyljodide sind durch die bekannte Telomerisationsreaktion von Perfluoralkyljodiden mit Äthylen leicht zugänglich. Diese Perfluoralkyljodide können reine Verbindungen sein oder aber Gemische von solchen Jodiden mit Perfluoralkyljodiden unterschiedlicher Kettenlänge. Letztere werden durch Telomerisation von kurzkettigen Perfluoralkyljodiden mit Tetrafluoräthylen erhalten, wobei gegebenenfalls das Ausgangsprodukt aus dem entstandenen Gemisch abgetrennt wird. Aus praktischen und wirtschaftlichen Gründen werden bevorzugt solche Gemische mit Äthylen telomerisiert und die so erhaltenen gemischtkettigen $R_f$-äthyljodide auch direkt in das erfindungsgemäße Verfahren als Ausgangsprodukte eingesetzt.

Die Ausgangs-$R_f$-äthyljodide entsprechen somit der allgemeinen Formel

$$R_f'(C_2F_4)_nC_2H_4J,$$

worin $R_f'$ Perfluormethyl, Perfluor-n-propyl, bevorzugt Perfluoräthyl und Perfluorisopropyl sowie n eine ganze Zahl oder im Falle von Gemischen auch eine gebrochene Zahl (als Mittelwert) von 0 bis 9 ist.

Das angewendete Molverhältnis von Wasser zu Jodid soll mindestens 1 : 1 betragen, ebenso das Molverhältnis von Methylpyrrolidon zu Jodid. Bei geringerer Wassermenge ist die Umsetzung der Jodide nicht vollständig. Wählt man eine zu große Wassermenge, wird eventuell die Selektivität der Umsetzung beeinträchtigt. Daher wird man im allgemeinen ein Molverhältnis von Wasser zu Jodid von 1 : 1 bis 10 : 1 anwenden. Vorzugsweise liegt dieses Molverhältnis zwischen 2 : 1 und 8 : 1.

Vorteilhaft setzt man N-Methyl-2-pyrrolidon im Überschuß ein. Vorzugsweise liegt daher das Molverhältnis von N-Methyl-2-pyrrolidon zu Jodid im Bereich von 2 : 1 bis 50 : 1, insbesondere zwischen 5 : 1 und 25 : 1. Größere Mengen von N-Methyl-2-pyrrolidon sind möglich, bringen aber keinen weiteren Vorteil.

Das erfindungsgemäße Verfahren wird bei Temperaturen von 100 bis 200 °C, vorzugsweise von 120 bis 160 °C, durchgeführt. Zweckmäßigerweise führt man die Umsetzung bei der Siedetemperatur des Ausgangsgemisches durch, die von der Zusammensetzung der $R_f$-äthyljodide und den Molverhältnissen der Reaktanten abhängt. Bei diesen Temperaturen ist die Umsetzung in etwa 8 bis 14 Stunden beendet.

Die Reaktion kann bei Normaldruck oder bei erhöhtem Druck durchgeführt werden. Zweckmäßigerweise werden die Reaktionsteilnehmer durch Rühren, Schütteln oder andere Durchmischungsmethoden intensiv miteinander vermischt.

Die erhaltenen $R_f$-äthanole werden durch Zugabe von Wasser zum Umsetzungsgemisch zusammen mit den als Nebenprodukt gebildeten $R_f$-äthylenen als untere Phase abgetrennt und gegebenenfalls mehrmals mit Wasser gewaschen.

Aus der unteren Phase werden die $R_f$-äthylene durch azeotrope Destillation mittels eines Alkanols mit 1 bis 8 C-Atomen oder eines Monoalkyläthers von Äthylenglykol als Schleppmittel abgetrennt, wie in der deutschen Patentanmeldung P 28 32 532.8 beschrieben. Anschließend können die $R_f$-äthanole im Vakuum destilliert werden.

Die erfindungsgemäß erhaltenen $R_f$-äthanole sind wertvolle Zwischenprodukte für die Herstellung von hydrophobierenden, oleophobierenden und/oder schmutzabweisenden Textilausrüstungsmitteln. Beispielsweise können aus diesen $R_f$-äthanolen deren Ester mit ungesättigten Carbonsäuren, beispielsweise mit Acryl- oder Methacrylsäure, erhalten werden, aus welchen durch Polymerisation technisch wichtige Produkte, insbesondere auf dem Gebiet der Textilausrüstung, gewonnen werden können.

Die folgenden Beispiele sollen das erfindungsgemäße Verfahren näher erläutern.

Beispiel 1

Man gibt in einen 2 l-Kolben, der mit Rührer und Thermometer ausgestattet ist, 272 g (0.5 Mol) $R_f$-äthyljodide mit der Kettenverteilung

$C_6F_{13}C_2H_4J$ 49.3 %
$C_8F_{17}C_2H_4J$ 32.8 %
$C_{10}F_{21}C_2H_4J$ 12.8 %
$C_{12}F_{25}C_2H_4J$ 5.1 %,

27 g (1.5 Mole) Wasser und 1.089 g (11 Mole) N-Methyl-2-pyrrolidon und erhitzt diese Mischung unter Rühren 13 Stunden auf 150 °C. Die entstandene Reaktionsmischung wird mit 2 l Wasser versetzt, wobei sich zwei Phasen bilden. Man wäscht die untere Phase zweimal mit je 500 ml Wasser 15 Minuten bei 70 °C.

Der Umsatz des $R_f$-äthyljodids beträgt 100 % (Bestimmung des Rest-Jodgehaltes mit Silbernitrat unter der Erfassungsgrenze), das Gaschromatogramm zeigt 91 % $R_f$-äthanole und 9 % $R_f$-äthylene.

Nach Zugabe von 500 ml Methanol zu der Mischung wird über eine 30 cm-Kolonne das die $R_f$-äthylene enthaltende Methanol abdestilliert bis zu einer Sumpftemperatur von 120 °C. Durch Versetzen des Destillates mit Wasser scheiden sich die $R_f$-äthylene als untere Phase ab.

Der verbleibende Kolbeninhalt wird ohne Kolonne weiterdestilliert ($Kp_{12}$ 70 bis 147 °C). Der Rückstand beträgt 3 %.

Das Gaschromatogramm des Destillates ergibt:

$R_f$-$C_2H_4OH$ 99.2 %
$R_f$-$CH = CH_2$ 0.8 %

Die Kettenverteilung entspricht der des eingesetzten $R_f$-äthyljodids.

Beispiel 2

Es wird die gleiche Apparatur wie in Beispiel 1 verwendet. Eine Mischung von

| | | |
|---|---|---|
| 237 g | $C_6F_{13}CH_2CH_2J$ | 0.5 Mol |
| 1 089 g | N-Methylpyrrolidon | 11.0 Mol |
| 27 g | $H_2O$ | 1.5 Mol |

wird 14 Stunden bei 140 °C gerührt. Der Reaktionsmischung wird zu Phasentrennung 1 l Wasser zugesetzt. Die untere Phase wird zweimal mit je 500 ml Wasser 15 Minuten bei 70 °C gewaschen. Eine gaschromatographische Analyse dieser unteren Phase ergibt eine vollständige Umsetzung des Jodids. Nach Zugabe von 500 ml Methanol in die gewaschene untere Phase und azeotroper Abdestillation von Methanol und $C_6F_{13}CH = CH_2$, wie in Beispiel 1 beschrieben, verbleibt als Rückstand $C_6F_{13}CH_2CH_2OH$, der ohne Kolonne weiterdestilliert wird. Dabei geht das $R_f$-äthanol über: $Kp_{12}$ 76 °C Menge 160.5 g = 0,44 Mol = 88 % Ausbeute; es verbleibt ein Rückstand von 5.5 g = 0.045 Mol = 3 %, der nach Elementaranalyse keinen Stickstoff enthält.

Aus dem Methanoldestillat wird durch Ausfällen mit Wasser 15.6 g $C_6F_{13}CH = CH_2$ = 0.045 Mol = 9 %, bezogen auf eingesetztes $R_f$-äthyljodid, erhalten.

Die gaschromatographische Analyse der Alkoholfraktion ergibt:

99.8 % $C_6F_{13}CH_2CH_2OH$
0.2 % $C_6F_{13}CH = CH_2$.

Beispiel 3

In einen 4 l-Kolben, mit Rührer und Thermometer und einer 30 cm Destillationskolonne ausgestattet, gibt man eine Mischung von

545 g $R_fCH_2CH_2J$ (1,0 Mol)

mit folgender Zusammensetzung

$C_6F_{13}CH_2CH_2J$ 42,3 %
$C_8F_{17}CH_2CH_2J$ 34,2 %
$C_{10}F_{21}CH_2CH_2J$ 17,5 %
$C_{12}F_{25}CH_2CH_2J$ 6,0 %,

ferner 594 g N-Methylpyrrolidon (6,0 Mol) und 72 g Wasser (4,0 Mol).

Man erhitzt unter Rühren 20 Stunden bis auf 142 °C. Dabei ist darauf zu achten, daß der niedersiedende Anteil des Reaktionsgemisches am Kolonnenkopf kräftig am Rückfluß kocht.

Die entstandene Reaktionsmischung wird mit 1 l Wasser versetzt und 1 Stunde bei 70 °C verrührt.

Nach dem Abstellen des Rührers trennen sich nach kurzer Zeit 520 g Rohgemisch als untere Phase ab.

Nach 2 maliger Wasserwäsche bei 70 °C wiegt das so gereinigte Reaktionsgemisch 438 g und hat folgende gaschromatographische Zusammensetzung :

$R_f$-CH = $CH_2$ 6,1 %
$R_f$-$CH_2CH_2OH$ 93,9 %

Nach Zugabe von 500 ml Methylglykol wird über eine 30 cm-Spiegelkolonne bis zu einer Sumpftemperatur von 140 °C das $R_f$-olefin/Methylglykol-Azeotrop abdestilliert.

Der zurückbleibende Rohalkohol wird ohne Kolonne über einen Claisenaufsatz bei 16 Torr im Bereich von 70 bis 147 °C überdestilliert.

Als Rückstand bleiben 7 g.

Das destillierte $R_f$-äthanol von 397,6 g hatte folgendes Gaschromatogramm :

$R_f$CH = $CH_2$ 0,3 %
$R_fCH_2CH_2OH$ 99,7 %

Die Kettenverteilung beim $R_fCH_2CH_2OH$ entspricht dem eingesetzten $R_f$-äthylenjodid.

**Ansprüche**

1. Verfahren zur Herstellung von 2-(Perfluoralkyl)-äthanolen der allgemeinen Formel

$R_fCH_2CH_2OH$,

worin $R_f$ einen geradkettigen oder endständig methylverzweigten Perfluoralkylrest mit 1 bis 21 Kohlenstoffatomen bedeutet, durch Umsetzung der entsprechenden 2-(Perfluoralkyl)-äthyljodide mit Wasser und Amiden bei Temperaturen von 100 bis 200 °C, dadurch gekennzeichnet, daß als Amid N-Methyl-2-pyrrolidon eingesetzt wird.

2. Verfahren nach Anspruch 1, dadurch gekennzeichnet, daß das Molverhältnis von Wasser zu 2-(Perfluoralkyl)-äthyljodid mindestens 1 : 1 beträgt.

3. Verfahren nach Anspruch 1, dadurch gekennzeichnet, daß das Molverhältnis von Wasser zu 2-(Perfluoralkyl)-äthyljodid 1 : 1 bis 10 : 1 beträgt.

4. Verfahren nach Anspruch 1 bis 3, dadurch gekennzeichnet, daß das Molverhältnis von N-Methyl-2-pyrrolidon zu 2-(Perfluoralkyl)-äthyljodid mindestens 1 : 1 beträgt.

5. Verfahren nach Anspruch 1 bis 3, dadurch gekennzeichnet, daß das Molverhältnis von N-Methyl-2-pyrrolidon zu 2-(Perfluoralkyl)-äthyljodid 2 : 1 bis 50 : 1 beträgt.

**Claims**

1. A process for the manufacture of 2-(perfluoroalkyl)-ethanols of the formula

$R_fCH_2CH_2OH$

wherein $R_f$ is a linear or terminally methyl-branched perfluoroalkyl having from 1 to 21 carbon atoms, from the corresponding 2-(perfluoroalkyl)-ethyl iodides by reacting said iodides with water and amides at a temperature in the range of from 100 to 200 °C, characterised in that the amid is N-methylpyrrolidone.

2. The process according to claim 1, characterised in that the molar ratio of water to 2-(perfluoroalkyl)-ethyl iodide is at least 1 : 1.

3. The process according to claim 1, characterised in that the molar ratio of water to 2-(perfluoroalkyl)-ethyl iodide is from 1 : 1 to 10 : 1.

4. The process according to claims 1 to 3, characterised in that the molar ratio of N-methyl-2-pyrrolidone to 2-(perfluoroalkyl)-ethyl iodide is at least 1 :1.

5. The process according to claims 1 to 3, characterised in that the molar ratio of N-methyl-2-pyrrolidone to 2-(perfluoroalkyl)-ethyl iodide is from 2 : 1 to 50 : 1.

**Revendications**

1. Procédé de préparation de (perfluoroalkyl)-2 éthanols répondant à la formule générale

$R_fCH_2CH_2OH$

dans laquelle $R_f$ représente un radical perfluoroalkyle contenant de 1 à 21 atomes de carbone, linéaire ou ramifié en bout de chaîne par un radical méthyle, par réaction des iodures de (perfluoroalkyl)-2 éthyles correspondants avec de l'eau et des amides, à des températures de 100 à 200 °C, procédé caractérisé en ce qu'on utilise, comme amide, la N-méthyl-pyrrolidone-2.

2. Procédé selon la revendication 1, caractérisé en ce que le rapport molaire de l'eau à l'iodure de (perfluoroalkyl)-2 éthyle est d'au moins 1 : 1.

3. Procédé selon la revendication 1, caractérisé en ce que le rapport molaire de l'eau à l'iodure de (perfluoroalkyl)-2 éthyle est compris entre 1 : 1 et 10 : 1.

4. Procédé selon l'une quelconque des revendications 1 à 3, caractérisé en ce que le rapport molaire de la N-méthyl-pyrrolidone-2 à l'iodure de (perfluoroalkyl)-2 éthyle est d'au moins 1 : 1.

5. Procédé selon l'une quelconque des revendications 1 à 3, caractérisé en ce que le rapport molaire de la N-méthyl-pyrrolidone-2 à l'iodure de (perfluoroalkyl)-2 éthyle est compris entre 2 : 1 et 50 : 1.